# EUROPEAN PATENT SPECIFICATION

(11) **EP 1 478 282 B1**
(45) Date of publication and mention of the grant of the patent: **16.06.2010**
(21) Application number: 02788088.9
(22) Date of filing: 10.12.2002
(51) Int. Cl.: A61B 17/02, A61F 2/08

(54) **SURGICAL DEVICE**
CHIRURGISCHE VORRICHTUNG
APPAREIL CHIRURGICAL

(30) Priority: 28.02.2002 GB 0204850
(43) Date of publication of application: 24.11.2004
(73) Proprietor: Campbell, Alexander, Craig, Wilhem, 42 Glasgow Road Kilsyth G65 9AD (GB)
(72) Inventor: Campbell, Alexander, Craig, Wilhem, 42 Glasgow Road Kilsyth G65 9AD (GB)
(74) Representative: Moreland, David
(86) International application number: PCT/GB2002/005582
(87) International publication number: WO 2003/071958

(56) References cited:
- WO-A-00/45691
- WO-A-01/62137
- GB-A- 2 097 260
- US-A- 4 733 850
- US-A- 5 061 245
- US-A- 5 531 232

## Description

### FIELD OF INVENTION

The present invention relates to a surgical device, and in particular, but not exclusively, to a surgical device for use in orthopaedics.

### BACKGROUND TO INVENTION

Tendon injuries can be difficult to successfully repair due to the nature in which tendons heal. Tendons are extremely sensitive to injury and physical intervention and are prone to considerable scarring and adhesion formation. Even puncture by a needle and suture or other surgical device can cause significant adhesion formation. The presence of scarring and adhesions can severely affect the movement and gliding of repaired tendons and are often the cause of failure in reconstruction procedures.

Of course, the presence of scarring will be determined by the type of injury and also by the method by which tendons are repaired. It is therefore preferred that relatively atraumatic reconstructive methods be used to minimise tissue reaction and scarring. Furthermore, it is also preferred that surgical intervention be minimised and repair be achieved in a single stage operation.

However, some tendon injuries such as long standing injuries to flexor tendons of the hand currently require a multi-stage operation, or a single stage operation which utilises harsh methods of repair, reducing the likelihood of success.

For example, a long-standing injury involving a severed flexor tendon of the hand will normally result in the tendon retracting from its tendon sheath. In this instance, during tendon grafting or reconstruction procedures the tendon sheath may require dilation which is conventionally achieved by a number of means, such as forcing metal bougies or probes into the sheath, along with other multipurpose instruments to allow sufficient sheath diameter for passage of a tendon graft or artificial material. Occasionally, however, and depending on the type of traumatising injury, the sheath may be too scarred or shrunken to allow this and the sheath has to be incised and refashioned/reattached to allow grafting. These methods normally involve a single stage operation and are relatively traumatic methods which may increase the risk of further scarring and an unsuccessful or non-gliding repair.

If the problem or injury is too severe for the above methods to be readily executed, then a multi stage operation may be required which may involve, for example, incising the sheath and implanting a silicone rod therein and allowing the tissue sufficient time to reform over the rod. Once the sheath has reformed, the tendon grafting procedure can be carried out.

PCT Patent WO 0162137 A (SPITZ, Gregory A) discloses devices for removing veins in a venous system of a patient. One device includes an elongated member having first and second lumens extending longitudinally therein. The first lumen extends from the proximal end of the elongated member to a first opening at the distal end of the member. The second lumen extends from the proximal end of the elongated member to a second opening in the side of the elongated member. The proximal end of the elongated member is coupled to a connector having two separate tubes that communicate with the respective first and second lumen for the injection and removal of fluid. A vein attachment member, attached to the elongated member, is adapted to be secured to the vein.

It is an object of at least one aspect of the present invention to obviate or at least mitigate the aforementioned problems in the prior art.

### SUMMARY OF INVENTION

According to a first aspect of the present invention, there is provided a surgical device according to claim 1.

The Applicant has conveniently called the device the "TENOCATH" (Trade Mark) device.

Although the surgical device of the present invention may be used for any purpose involving the requirement to dilate tissue, the device is preferably a tendon reconstructive surgery device, such as a hand flexor tendon repair surgery device.

Advantageously, the surgical device may be a single-stage operation tendon repair surgery device.

Preferably, the balloon is inflated with a sterile fluid injected into the central bore of the body portion and through the at least one perforation using a syringe coupled thereto.

Preferably also, the balloon is deflated by elastic recovery of the balloon material which forces the sterile fluid out of the balloon and into the central bore of the body portion via the at least one perforation disposed on the outer surface thereof.

Conveniently, the elongate body portion of the device is flexible.

Preferably, the body portion is made of a material having a low frictional coefficient to facilitate easy passage through a tendon sheath or the like.

The body portion may be made of a plastics material such as polyvinyl chloride (PVC) or other plastic of medical grade (United States Plastic (USP) grade IV or VI).

In a preferred embodiment, the means for receiving a syringe is a luer lock having an outer diameter greater than that of the body portion and having a throughbore coaxially aligned with the central bore of the body portion.

Advantageously, the luer lock comprises flange portions allowing the luer lock to be gripped by the syringe while the syringe is inserted and used therein. The syringe may be a conventional syringe, or alternatively, may comprise a threaded collar which is adapted to engage a corresponding threaded portion on the luer lock such that the syringe may be coupled thereto.

Preferably, the balloon is manufactured from latex, or alternatively from any other suitable resilient material including silicone rubber.

Conveniently, the balloon may have a fluid capacity of between 0.05 ml to 1.1 ml.

Preferably, the balloon has a wall thickness of approximately 0.3 mm thick.

Advantageously, the outer diameter of the portion of the body around which the balloon is disposed may be reduced such that the body portion has a substantially constant outer diameter when the balloon is deflated. This allows for smooth passage of the device through a tendon sheath, for example, and prevents damage of the balloon when the device is not in use.

In a preferred embodiment of the present invention, the body portion is tapered from the open end to the closed end thereof. This allows the device to be more easily inserted into a tendon sheath, for example.

Preferably, the device is sterile and is provided in a hermetic packaging to prevent contamination until such time as the device is required.

Preferably, the device has a length of approximately 0.5 m or less. More preferably, the device has a length of approximately 0.25 m.

Preferably, the body portion has an outer diameter of approximately 2 mm.

The body portion may have an inner diameter of approximately 1 mm or less and preferably between 0.5mm to 1mm.

Preferably, the portion of the body portion around which the balloon is disposed has an outer diameter of approximately 1.6 mm.

Preferably also, the length of the body portion around which the balloon is disposed is approximately 10 mm for a balloon capacity of approximately 1 ml.

Preferably, the luer lock has a length of approximately 20 mm and an outer diameter ranging from 6 mm at an open end to 5 mm at the end coupled to the body portion. Preferably also, the luer lock throughbore has an open diameter of approximately 4.4 mm. Additionally, preferably the flange portion of the luer lock has an outer diameter of approximately 7.5 mm.

Preferably, the aperture provided in the closed end of the device has a diameter of approximately 0.7 mm.

The surgical device may have means for dilating an orifice in a body part and means for engaging an elongate member, wherein the dilating means comprise the deflatably inflatable balloon mounted on the body portion, the engaging means comprising the aperture on the body portion, said aperture adapted for receiving the suture, and the balloon being partially detachable from the body portion so as to be capable of being drawn over the aperture.

Conveniently, the body part is a tendon sheath.

Conveniently also, the elongate member is a flexible cord-like structure.

The cord-like structure may be a retrieved tendons, a tendon graft or an artificial tendon material or the like.

Thus, the device of the present invention may be used as both a dilator and a passer.

In particular the device may be a tendon reconstruction surgical device.

The dilating means may comprise a deflatably inflatable balloon, and the engaging means may comprise an eye for suturing the tendon or suitable artificial material to the device.

The balloon may be mounted on the body portion at two points of attachment, the balloon being detachable from the body portion at an attachment point furthest removed from the aperture.

### BRIEF DESCRIPTION OF DRAWINGS

These and other aspects of the present invention will now be described, by way of example only, with reference to the accompanying drawings in which:
Figure 1 is a side view of a surgical device in accordance with a preferred embodiment of the present invention;
Figure 2 is an enlarged view of an end portion of the device of Figure 1;
Figure 3 is an end view of another end of the device of Figure 1;
Figures 4 and 5 show the device of Figure 1 in use; and
Figure 6 and 7 show the device of Figure 1 in use in a surgical procedure to repair a divided tendon of the hand.

### DETAILED DESCRIPTION OF DRAWINGS

While it would be clear to a person of skill in the art that the surgical device of the present invention may be used in a number of procedures, in the interests of brevity and clarity, the following description of prefered embodiments relates to a surgical device particularly advantageously for use in tendon surgery.

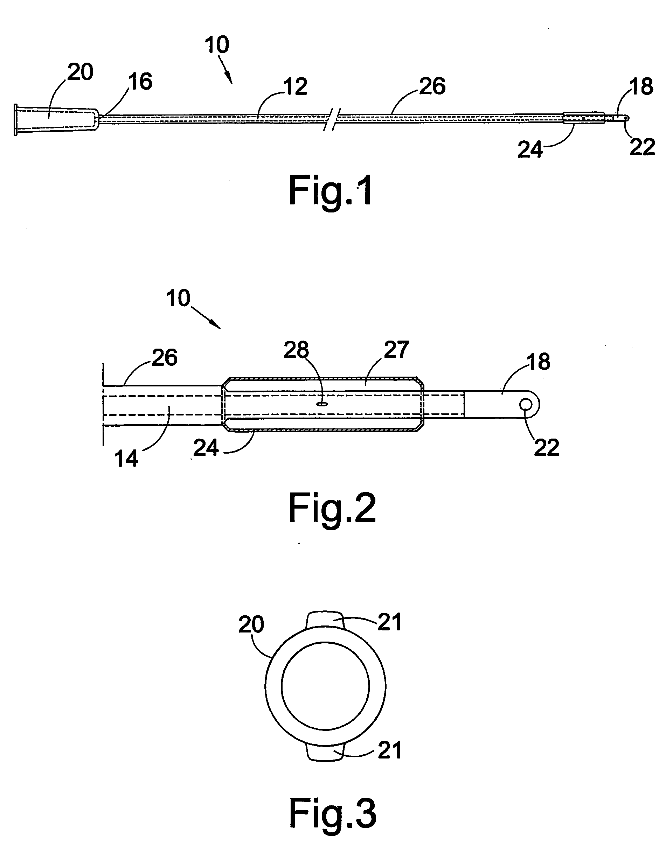

Reference is first made to Figures 1 and 2 of the drawings in which there is shown a surgical device 10 in accordance with a preferred embodiment of the present invention, wherein Figure 2 is an enlarged view of an end portion of the device shown in Figure 1.

The device 10 comprises a plastic flexible elongate body portion 12 which has a central bore 14 (Figure 2) which extends from an open end 16 to a closed end 18 of the body portion 12. Coupled to the open end 16 is a luer lock 20 for recieving a syringe (not shown). The luer lock 20 has an outer diameter greater than that of the body portion 12 and has a throughbore (not shown) coaxially aligned with the central bore 14 of the body portion 12. Additionally, the luer lock 20 comprises flange portions 21, shown in Figure 3, allowing the luer lock to be gripped by the collar of a syringe while a syringe is inserted and used therein.

An aperture 22 is provided in the closed end 18 of the body portion 12, wherein the aperture 22 is adapted for receiving a suture to attach a tendon or other suitable artificial material to the device 10.

Disposed around a portion 27 of the body portion 12 in proximity to the closed end 18 thereof is a latex balloon 24 which forms a fluid tight seal with the outer surface 26 of the body portion 12 and is in fluid communication with the central bore 14 via perforations 28 (only one shown in Figure 2) disposed on the outer surface 26 of the body portion 12.

As clearly shown in Figure 2, the outer diameter of the portion of the body 12 around which the balloon 24 is disposed is reduced such that the body portion 12 has a substantially constant outer diameter when deflated. This allows the device to be easily passed through tendon sheaths, for example.

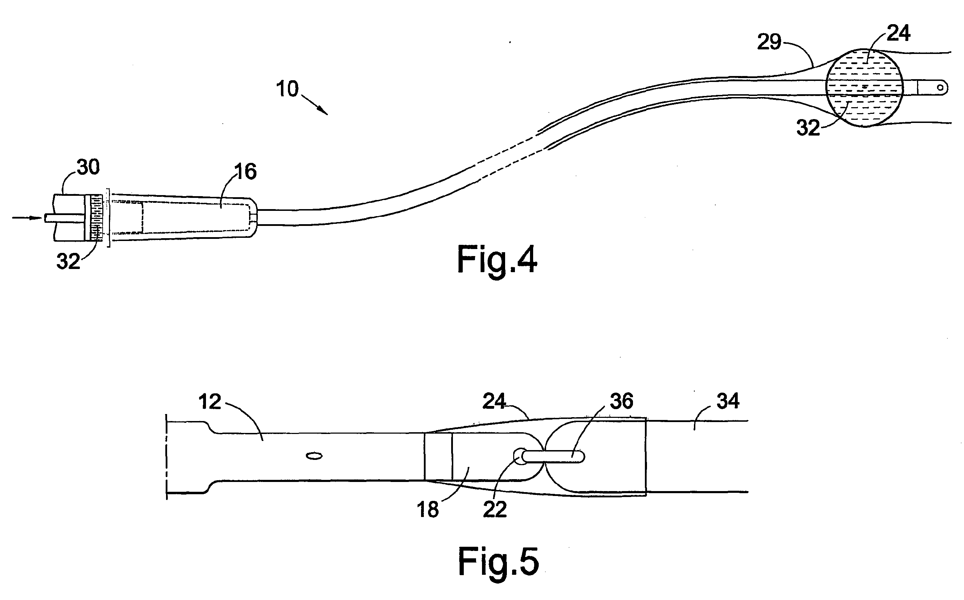

Reference is now made to Figure 4 in which there is shown the device 10 of Figure 1 in use. The device 10 is located within a tendon sheath 29, and a syringe 30 containing a sterile fluid 32 such as saline solution is coupled to the luer lock 16. The sterile fluid 32 is injected into the device through the central bore 14 (not shown) and into the balloon 24 causing the balloon to inflated in order to dilate the tendon sheath 29. To dilate the entire length of the sheath 29, the device 10 is progressively retracted and the balloon 24 reinflated.

Once the entire sheath has been dilated to the required extent, the device is passed therethrough and a tendon graft 34, for example, is fixed to the aperture 22 with a suture 36, as shown in Figure 5. One end of the balloon 24 is detached from the body portion 12 and is drawn over the aperture 22 in the closed end 18 to provide a smooth interface between the device 10 and the tendon graft 34. This protects the tendon graft 34 and prevents it from becoming detached from the device 10 when the device is retracted from the sheath in order to pull the tendon graft 34 therethrough.

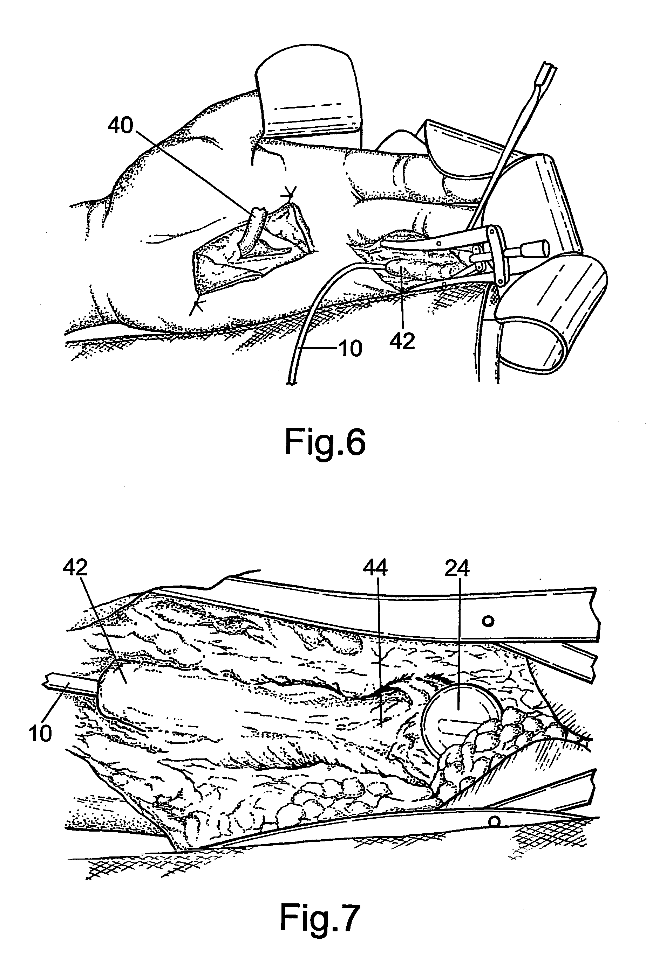

Reference is now made to Figures 6 and 7 of the drawings in which there is shown the device 10 of the present invention in use in a surgical procedure to repair a divided flexor digitorum profundus tendon of the hand. In this particular case, the tendon has become detached from the distal phalanx of the ring finger and the tendon stump 40 has become retracted from its synovial sheath 42, resulting in contraction of the sheath 42.

The sheath 42 has been exposed and the device 10 has been inserted therein from proximally to distally, that is, from the end nearest the palmar surface of the hand to the top of the finger. As shown in Figure 7, the device 10 is fully inserted to reveal the balloon 24, which has been inflated for clarity. The operative technique then involves aligning the balloon with the distal end 44 of the sheath 42 and inflating to its maximum capacity of approximately 1 ml, and retaining the balloon 24 in this state for approximately 1 minute to allow the tissue to equilibrate. The balloon 24 is then deflated and the device retracted by one or two centimetres, and the process repeated until the entire length of the sheath 42 has been sufficiently dilated. Once completed, the balloon 24 may be semi-inflated and passed through the sheath 42 to check adequate dilation.

The stump of the retracted tendon 40 is then isolated and palmaris longus tendon, for example, is harvested to be used as a tendon graft. The distal end of the graft is then anchored to the base of the distal phalanx using a pullout suture technique over a dental roll, for example. The graft is then sutured to the aperture 22 in the device which is proximally pulled through the dilated sheath 42. The proximal end of the tendon graft is sutured to the profundus stump using an interlacing technique, for example.

Thus, using the device 10 of the present invention in the manner discussed above allows a relatively atraumatic, one stage reconstruction process to be carried out which increases the likelihood of successful repair.

It would be apparent to a person of skill in the art that the embodiments hereinbefore described are merely exemplary of the present invention and various modifications may be made thereto without departing from the scope of the invention. For example, the device is particularly beneficial in but not limited for use in orthopaedics. The device is most advantageously used in tendon surgery, however the novel devices of the second and third aspects may be used in other surgical fields where the requirement for the dilation of tissue exists. Further, the device may be used in tendon harvesting procedures. The body portion may be manufactured from any suitable flexible material. Additionally, the balloon may be made of any suitable resilient material. The dimensions may be selected in accordance with the required use of the device.

## Claims

1. A surgical device (10) comprising:
an elongate body portion (12) having a central bore (14) extending from an open end (16) to a closed end (18) thereof;
means for receiving a syringe (30), said means coupled to the open end of the body portion;
at least one aperture (22) in the closed end of the body portion, said at least one aperture adapted for receiving a suture (36); and
an inflatable balloon (24) disposed around a portion (27) of the body portion in proximity to the closed end thereof, said balloon forming a fluid tight seal with the outer surface (26) of the body portion and being in fluid communication with the central bore via at least one perforation (28) disposed on the outer surface of the body portion, wherein the balloon is detachable from the body portion at the side nearest the open end thereof so as to be capable of being drawn over the aperture.

2. A surgical device as claimed in claim 1, wherein the surgical device is a tendon reconstruction surgery device.

3. A surgical device as claimed in claim 1 or 2, wherein the surgical device is a hand flexor tendon repair surgery device.

4. A surgical device as claimed in any one of claims 1, 2 or 3, wherein the surgical device is a single-stage operation tendon repair surgery device.

5. A surgical device as claimed in any one of claims 1 to 4, wherein the balloon is inflatable with a sterile fluid (32) injected into the central bore of the body portion and through the at least one perforation using a syringe coupled thereto.

6. A surgical device as claimed in claim 5, wherein the balloon is deflatable by elastic recovery of the balloon material which forces the sterile fluid out of the balloon and into the central bore of the body portion via the at least one perforation disposed on the outer surface thereof.

7. A surgical device as claimed in any one of claims 1 to 6, wherein the elongate body portion of the device is flexible.

8. A surgical device as claimed in any one of claims 1 to 7, wherein the body portion is made of a material having a low frictional coefficient to facilitate easy passage through a tendon sheath (29).

9. A surgical device as claimed in any one of claims 1 to 8, wherein the body portion is made of a plastics material.

10. A surgical device as claimed in any one of claims 1 to 9, wherein the body portion is made of polyvinyl chloride (PVC).

11. A surgical device as claimed in any one of claims 1 to 10, wherein the body portion is made of a plastic of medical grade, being United States plastic (USP) grade IV or VI.

12. A surgical device as claimed in any one of claims 1 to 11, wherein the means for receiving a syringe is a luer lock (20) having an outer diameter greater than that of the body portion and having a throughbore coaxially aligned with the central bore of the body portion.

13. A surgical device as claimed in claim 12, wherein the luer lock comprises flange portions (21) allowing the luer lock to be gripped by the syringe while the syringe is inserted and used therein.

14. A surgical device as claimed in any one of claims 1 to 11, wherein the means for receiving a syringe comprises a threaded portion for engaging a threaded collar mounted on a syringe.

15. A surgical device as claimed in any one of claims 1 to 14, wherein the balloon is manufactured from a resilient material.

16. A surgical device as claimed in any one of claims 1 to 15, wherein the balloon is manufactured from latex.

17. A surgical device as claimed in any one of claims 1 to 16, wherein the balloon has a fluid capacity of between 0.05 ml to 1.1 ml.

18. A surgical device as claimed in any one of claims 1 to 17, wherein the balloon has a wall thickness of approximately 0.3 mm.

19. A surgical device as claimed in any one of claims 1 to 18, wherein the outer diameter of the portion of the body around which the balloon is disposed is reduced such that the body portion has a substantially constant outer diameter when the balloon is deflated.

20. A surgical device as claimed in any one of claims 1 to 19, wherein the body portion is tapered from the open end to the closed end thereof.

21. A surgical device as claimed in any one of claims 1 to 20, wherein the device is sterile and is provided in a hermetic packaging to prevent contamination until such time as the device is required.

22. A surgical device as claimed in any one of claims 1 to 21, wherein the device has a length of approximately 0.5m or less.

23. A surgical device as claimed in any one of claims 1 to 21, wherein the device has a length of approximately 0.25 m.

24. A surgical device as claimed in any one of claims 1 to 23, wherein the body portion has an outer diameter of approximately 2 mm.

25. A surgical device as claimed in any one of claims 1 to 24, wherein the body portion has an inner diameter of approximately 1 mm or less.

26. A surgical device as claimed in any one of claims 1 to 24, wherein the body portion has an inner diameter of between 0.5mm to 1mm.

27. A surgical device as claimed in any one of claims 1 to 26, wherein the portion of the body portion around which the balloon is disposed has an outer diameter of approximately 1.6 mm.

28. A surgical device as claimed in any one of claims 1 to 27, wherein the length of the body portion around which the balloon is disposed is approximately 10 mm for a balloon capacity of approximately 1 ml.

29. A surgical device as claimed in claim 12 or 13, wherein the luer lock has a length of approximately 20 mm and an outer diameter ranging from 6 mm at an open end to 5 mm at the end coupled to the body portion.

30. A surgical device as claimed in claim 12, 13 or 29, wherein the luer lock throughbore has an open diameter of approximately 4.4 mm.

31. A surgical device as claimed in claim 12, 13, 29 or 30, wherein the flange portion of the luer lock has an outer diameter of approximately 7.5 mm.

32. A surgical device as claimed in any one of claims 1 to 31, wherein the aperture provided in the closed end of the device has a diameter of approximately 0.7 mm.

33. A surgical device (10) as claimed in claim 1, having means for dilating an orifice in a body part and means for engaging an elongate member, wherein the dilating means comprises the deflatably inflatable balloon (24) mounted on the body portion (12), the engaging means comprising the aperture (22) on the body portion, said aperture adapted for receiving the suture (36), and the balloon being partially detachable from the body portion so as to be capable of being drawn over the aperture.

34. A surgical device as claimed in claim 33, wherein the body part is a tendon sheath (29).

35. A surgical device as claimed in claim 33 or 34, wherein the elongate member is a flexible cord-like structure (34).

36. A surgical device as claimed in claim 35, wherein the cord-like structure is a retrieved tendon.

37. A surgical device as claimed in claim 35, wherein the cord-like structure is a tendon graft.

38. A surgical device as claimed in claim 35, wherein the cord-like structure is an artificial tendon material.

39. A surgical device as claimed in any of claims 33 to 38, wherein the surgical device is both a dilating and passing surgical device.

40. A surgical device as claimed in any one of claims 33 to 39, wherein the surgical device is a tendon reconstruction surgical device.

41. A surgical device as claimed in any one of claims 33 to 40, wherein the balloon is mounted on the body portion at two points of attachment, the balloon being detachable from the body portion at an attachment point furthest removed from the aperture.

## Patentansprüche

1. Chirurgische Vorrichtung (10), die aufweist:
einen länglichen Körperabschnitt (12) mit einer mittigen Bohrung (14), die sich von dessen einem offenen Ende (16) zu einem geschlossenen Ende (18) erstreckt;
eine Einrichtung für das Aufnehmen einer Spritze (30), wobei die Einrichtung mit dem offenen Ende des Körperabschnittes verbunden ist;
mindestens eine Öffnung (22) im geschlossenen Ende des Körperabschnittes, wobei die mindestens eine Öffnung für das Aufnehmen eines Nahtmaterials (36) ausgebildet ist; und
einen aufblasbaren Ballon (24), der um einen Teil (27) des Körperabschnittes in unmittelbarer Nähe zu dessen geschlossenem Ende angeordnet ist, wobei der Ballon eine fluiddichte Dichtung mit der äußeren Fläche (26) des Körperabschnittes bildet und in Fluidverbindung mit der mittigen Bohrung über mindestens eine Perforation (28) ist, die auf der äußeren Fläche des Körperabschnittes angeordnet ist, wobei der Ballon vom Körperabschnitt an der Seite abnehmbar ist, die dessen offenem Ende am nächsten ist, damit ein Ziehen über die Öffnung erfolgen kann.

2. Chirurgische Vorrichtung nach Anspruch 1, bei der die chirurgische Vorrichtung eine chirurgische Vorrichtung für die Sehnenrekonstruktion ist.

3. Chirurgische Vorrichtung nach Anspruch 1 oder Anspruch 2, bei der die chirurgische Vorrichtung eine chirurgische Vorrichtung für die Wiederherstellung einer Handbeugesehne ist.

4. Chirurgische Vorrichtung nach einem der Ansprüche 1, 2 oder 3, bei der die chirurgische Vorrichtung eine chirurgische Vorrichtung für die Sehnenwiederherstellung in einer einzigen Operationsstufe ist.

5. Chirurgische Vorrichtung nach einem der Ansprüche 1 bis 4, bei der der Ballon mit einem sterilen Fluid (32) aufblasbar ist, das in die mittige Bohrung des Körperabschnittes und durch die mindestens eine Perforation bei Benutzung einer damit verbundenen Spritze injiziert wird.

6. Chirurgische Vorrichtung nach Anspruch 5, bei der der Ballon durch Rückverformung des Ballonmaterials entleert werden kann, das das sterile Fluid aus dem Ballon heraus und in die mittige Bohrung des Körperabschnittes hinein über die mindestens eine in dessen äußerer Fläche angeordnete Perforation drückt.

7. Chirurgische Vorrichtung nach einem der Ansprüche 1 bis 6, bei der der längliche Körperabschnitt der Vorrichtung flexibel ist.

8. Chirurgische Vorrichtung nach einem der Ansprüche 1 bis 7, bei der der Körperabschnitt aus einem Material mit einem niedrigen Reibungskoeffizienten besteht, um einen leichten Durchgang durch die Sehnenscheide (29) zu begünstigen.

9. Chirurgische Vorrichtung nach einem der Ansprüche I bis 8, bei der der Körperabschnitt aus einem Kunststoffmaterial besteht.

10. Chirurgische Vorrichtung nach einem der Ansprüche 1 bis 9, bei der der Körperabschnitt aus Polyvinylchlorid (PVC) besteht.

11. Chirurgische Vorrichtung nach einem der Ansprüche 1 bis 10, bei der der Körperabschnitt aus einem medizinischen Kunststoff besteht, der ein United States Plastics (USP) Güteklasse IV oder VI ist.

12. Chirurgische Vorrichtung nach einem der Ansprüche 1 bis 11, bei der die Einrichtung für das Aufnehmen einer Spritze ein Luer-Verschluss (20) mit einem Außendurchmesser ist, der größer ist als der des Körperabschnittes, und der eine Durchgangsbohrung aufweist, die mit der mittigen Bohrung des Körperabschnittes koaxial ausgerichtet ist.

13. Chirurgische Vorrichtung nach Anspruch 12, bei der der Luer-Verschluss Flanschabschnitte (21) aufweist, die gestatten, dass der Luer-Verschluss durch die Spritze ergriffen wird, während die Spritze darin eingesetzt und verwendet wird.

14. Chirurgische Vorrichtung nach einem der Ansprüche 1 bis 11, bei der die Einrichtung für das Aufnehmen einer Spritze einen Gewindeabschnitt für einen Eingriff mit einem Gewindering aufweist, der auf einer Spritze montiert ist.

15. Chirurgische Vorrichtung nach einem der Ansprüche 1 bis 14, bei der der Ballon aus einem elastischen Material hergestellt wird.

16. Chirurgische Vorrichtung nach einem der Ansprüche 1 bis 15, bei der der Ballon aus Latex hergestellt wird.

17. Chirurgische Vorrichtung nach einem der Ansprüche 1 bis 16, bei der der Ballon ein Fluidfassungsvermögen zwischen 0,05 ml und 1,1 ml aufweist.

18. Chirurgische Vorrichtung nach einem der Ansprüche 1 bis 17, bei der der Ballon eine Wanddicke von annähernd 0,3 mm aufweist.

19. Chirurgische Vorrichtung nach einem der Ansprüche 1 bis 18, bei der der Außendurchmesser des Abschnittes des Körpers, um den der Ballon herum angeordnet wird, so verringert ist, dass der Körperabschnitt einen im Wesentlichen konstanten Außendurchmesser aufweist, wenn der Ballon entleert ist.

20. Chirurgische Vorrichtung nach einem der Ansprüche 1 bis 19, bei der sich der Körperabschnitt von dessen offenem Ende zu dessen geschlossenem Ende verjüngt.

21. Chirurgische Vorrichtung nach einem der Ansprüche 1 bis 20, bei der die Vorrichtung steril ist und in einer hermetischen Verpackung bereitgestellt wird, um eine Verunreinigung bis zu einem Zeitpunkt zu verhindern, zu dem die Vorrichtung benötigt wird.

22. Chirurgische Vorrichtung nach einem der Ansprüche 1 bis 21, bei der die Vorrichtung eine Länge von annähernd 0,5 m oder weniger aufweist.

23. Chirurgische Vorrichtung nach einem der Ansprüche 1 bis 21, bei der die Vorrichtung eine Länge von annähernd 0,25 m aufweist.

24. Chirurgische Vorrichtung nach einem der Ansprüche 1 bis 23, bei der der Körperabschnitt einen Außendurchmesser von annähernd 2 mm aufweist.

25. Chirurgische Vorrichtung nach einem der Ansprüche 1 bis 24, bei der der Körperabschnitt einen Innendurchmesser von annähernd 1 mm oder weniger aufweist.

26. Chirurgische Vorrichtung nach einem der Ansprüche 1 bis 24, bei der der Körperabschnitt einen Inndurchmesser zwischen 0,5 mm und 1 mm aufweist.

27. Chirurgische Vorrichtung nach einem der Ansprüche 1 bis 26, bei der der Teil des Körperabschnittes, um den der Ballon herum angeordnet ist, einen Außendurchmesser von annähernd 1,6 mm aufweist.

28. Chirurgische Vorrichtung nach einem der Ansprüche 1 bis 27, bei der die Länge des Körperabschnittes, um den der Ballon herum angeordnet ist, annähernd 10 mm für ein Ballonfassungsvermögen von annähernd 1 ml aufweist.

29. Chirurgische Vorrichtung nach Anspruch 12 oder 13, bei der der Luer-Verschluss eine Länge von annähernd 20 mm und einen Außendurchmesser aufweist, der sich von 6 mm an einem offenen Ende bis zu 5 mm an dem Ende bewegt, das mit dem Körperabschnitt verbunden ist.

30. Chirurgische Vorrichtung nach Anspruch 12, 13 oder 29, bei der die Luer-Verschlussdurchgangsbohrung einen offenen Durchmesser von annähernd 4,4 mm aufweist.

31. Chirurgische Vorrichtung nach Anspruch 12, 13, 29 oder 30, bei der der Flanschabschnitt des Luer-Verschlusses einen Außendurchmesser von annähernd 7,5 mm aufweist.

32. Chirurgische Vorrichtung nach einem der Ansprüche 1 bis 31, bei der die im geschlossenen Ende der Vorrichtung vorhandene Öffnung einen Durchmesser von annähernd 0,7 mm aufweist.

33. Chirurgische Vorrichtung (10) nach Anspruch 1, die eine Einrichtung für das Aufweiten einer Öffnung in einem Körperteil und eine Einrichtung für das Ineingriffkommen mit einem länglichen Element aufweist, wobei die Einrichtung für das Aufweiten den entleerbar aufblasbaren Ballon (24) aufweist, der am Körperabschnitt (12) montiert ist, wobei die Einrichtung für das Ineingriffkommen die Öffnung (22) im Körperabschnitt aufweist, wobei die Öffnung für das Aufnehmen des Nahtmaterials (36) ausgebildet ist, und wobei der Ballon teilweise vom Körperabschnitt abnehmbar ist, damit ein Ziehen über die Öffnung erfolgen kann.

34. Chirurgische Vorrichtung nach Anspruch 33, bei der das Körperteil eine Sehnenscheide (29) ist.

35. Chirurgische Vorrichtung nach Anspruch 33 oder 34, bei der das längliche Element eine flexible schnurartige Struktur (34) ist.

36. Chirurgische Vorrichtung nach Anspruch 35, bei der die schnurartige Struktur eine zurückgewonnene Sehne ist.

37. Chirurgische Vorrichtung nach Anspruch 35, bei der die schnurartige Struktur ein Sehnenimplantat ist.

38. Chirurgische Vorrichtung nach Anspruch 35, bei der die schnurartige Struktur ein künstliches Sehnenmaterial ist.

39. Chirurgische Vorrichtung nach einem der Ansprüche 33 bis 38, bei der die chirurgische Vorrichtung sowohl eine aufweitende als auch hindurchgehende chirurgische Vorrichtung ist.

40. Chirurgische Vorrichtung nach einem der Ansprüche 33 bis 39, bei der die chirurgische Vorrichtung eine chirurgische Vorrichtung für die Sehnenrekonstruktion ist.

41. Chirurgische Vorrichtung nach einem der Ansprüche 33 bis 40, bei der der Ballon am Körperabschnitt an zwei Befestigungsstellen montiert ist, wobei der Ballon vom Körperabschnitt an einer Befestigungsstelle abnehmbar ist, die von der Öffnung am weitesten entfernt ist.

## Revendications

1. Dispositif chirurgical (10), comprenant :
une partie de corps allongée (12), comportant un alésage central (14) s'étendant de son extrémité ouverte (16) vers son extrémité fermée (18) ;
un moyen pour recevoir une seringue (30), ledit moyen étant accouplé à l'extrémité ouverte de la partie de corps ;
au moins une ouverture (22) dans l'extrémité fermée de la partie de corps, ladite au moins une ouverture étant adaptée pour recevoir un fil de suture (36) ; et
un ballonnet gonflable (24) agencé autour d'une partie (27) de la partie de corps, à proximité de son extrémité fermée, ledit ballonnet établissant un joint étanche au fluide avec la surface externe (26) de la partie de corps et étant en communication de fluide avec l'alésage central par l'intermédiaire d'au moins une perforation (28) agencée sur la surface externe de la partie de corps, le ballonnet pouvant être détaché de la partie de corps au niveau du côté le plus proche de son extrémité ouverte, de sorte à pouvoir être tiré au-dessus de l'ouverture.

2. Dispositif chirurgical selon la revendication 1, dans lequel le dispositif chirurgical est un dispositif de chirurgie de reconstruction d'un tendon.

3. Dispositif chirurgical selon les revendications 1 ou 2, dans lequel le dispositif chirurgical est un dispositif de chirurgie réparatrice d'un tendon fléchisseur de la main.

4. Dispositif chirurgical selon l'une quelconque des revendications 1, 2 ou 3, dans lequel le dispositif chirurgical est un dispositif de chirurgie réparatrice d'un tendon dans le cadre d'une opération à intervention unique.

5. Dispositif chirurgical selon l'une quelconque des revendications 1 à 4, dans lequel le ballonnet peut être gonflé avec un fluide stérile (32) injecté dans l'alésage central de la partie de corps et à travers la au moins une perforation par l'intermédiaire d'une seringue qui y est accouplée.

6. Dispositif chirurgical selon la revendication 5, dans lequel le ballonnet peut être dégonflé par reprise élastique du matériau du ballonnet, entraînant le fluide stérile hors du ballonnet et dans l'alésage central de la partie de corps à travers la au moins une perforation agencée sur sa surface externe.

7. Dispositif chirurgical selon l'une quelconque des revendications 1 à 6, dans lequel la partie de corps allongée du dispositif est flexible.

8. Dispositif chirurgical selon l'une quelconque des revendications 1 à 7, dans lequel la partie de corps est composée d'un matériau à coefficient de frottement réduit pour assurer un passage facile à travers une gaine tendineuse (29).

9. Dispositif chirurgical selon l'une quelconque des revendications 1 à 8, dans lequel la partie de corps est composée d'un matériau plastique.

10. Dispositif chirurgical selon l'une quelconque des revendications 1 à 9, dans lequel la partie de corps est composée de chlorure de polyvinyle (PVC).

11. Dispositif chirurgical selon l'une quelconque des revendications 1 à 10, dans lequel la partie de corps est composée d'un plastique de qualité médicale, à savoir de qualité IV ou VI selon la norme « United States Plastic (USP) ».

12. Dispositif chirurgical selon l'une quelconque des revendications 1 à 11, dans lequel le moyen destiné à recevoir une seringue est une vis Luer (20), ayant un diamètre extérieur supérieur à celui de la partie de corps et comportant un alésage de passage aligné de manière coaxiale avec l'alésage central de la partie de corps.

13. Dispositif chirurgical selon la revendication 12, dans lequel la vis Luer comprend des parties de bride (21), permettant la saisie de la vis Luer par la seringue pendant l'insertion de la seringue et son utilisation dans le dispositif.

14. Dispositif chirurgical selon l'une quelconque des revendications 1 à 11, dans lequel le moyen destiné à recevoir une seringue comprend une partie filetée destinée à s'engager dans un collet fileté monté sur une seringue.

15. Dispositif chirurgical selon l'une quelconque des revendications 1 à 14, dans lequel le ballonnet est fabriqué à partir d'un matériau élastique.

16. Dispositif chirurgical selon l'une quelconque des revendications 1 à 15, dans lequel le ballonnet est fabriqué à partir de latex.

17. Dispositif chirurgical selon l'une quelconque des revendications 1 à 16, dans lequel le ballonnet a une capacité de fluide comprise entre 0,05 ml et 1,1 ml.

18. Dispositif chirurgical selon l'une quelconque des revendications 1 à 17, dans lequel le ballonnet a une épaisseur de paroi d'environ 0,3 mm.

19. Dispositif chirurgical selon l'une quelconque des revendications 1 à 18, dans lequel le diamètre extérieur de la partie de corps autour de laquelle le ballonnet est agencé est réduit, de sorte que la partie de corps a un diamètre extérieur pratiquement constant lorsque le ballonnet est dégonflé.

20. Dispositif chirurgical selon l'une quelconque des revendications 1 à 19, dans lequel la partie de corps est effilée de son extrémité ouverte vers son extrémité fermée.

21. Dispositif chirurgical selon l'une quelconque des revendications 1 à 20, dans lequel le dispositif est stérile et est fourni dans un emballage hermétique pour empêcher une contamination jusqu'au moment où le dispositif est utilisé.

22. Dispositif chirurgical selon l'une quelconque des revendications 1 à 21, dans lequel le dispositif a une longueur d'environ 0,5 m ou moins.

23. Dispositif chirurgical selon l'une quelconque des revendications 1 à 21, dans lequel le dispositif a une longueur d'environ 0,25 m.

24. Dispositif chirurgical selon l'une quelconque des revendications 1 à 23, dans lequel la partie de corps a un diamètre extérieur d'environ 2 mm.

25. Dispositif chirurgical selon l'une quelconque des revendications 1 à 24, dans lequel la partie de corps a un diamètre intérieur d'environ 1 mm ou moins.

26. Dispositif chirurgical selon l'une quelconque des revendications 1 à 24, dans lequel la partie de corps a un diamètre intérieur compris entre 0,5 mm et 1 mm.

27. Dispositif chirurgical selon l'une quelconque des revendications 1 à 26, dans lequel la partie de la partie de corps autour de laquelle est agencé le ballonnet a un diamètre extérieur d'environ 1,6 mm.

28. Dispositif chirurgical selon l'une quelconque des revendications 1 à 27, dans lequel la longueur de la partie de corps autour de laquelle est agencé le ballonnet correspond à environ 10 mm pour une capacité du ballonnet d'environ 1 ml.

29. Dispositif chirurgical selon les revendications 12 ou 13, dans lequel la vis Luer a une longueur d'environ 20 mm et un diamètre extérieur compris entre 6 mm au niveau d'une extrémité ouverte et 5 mm au niveau de l'extrémité accouplée à la partie de corps.

30. Dispositif chirurgical selon les revendications 12, 13 ou 29, dans lequel l'alésage de passage de la vis Luer a un diamètre ouvert d'environ 4,4 mm.

31. Dispositif chirurgical selon les revendications 12, 13, 29 ou 30, dans lequel la partie de bride de la vis Luer a un diamètre extérieur d'environ 7,5 mm.

32. Dispositif chirurgical selon l'une quelconque des revendications 1 à 31, dans lequel l'ouverture formée dans l'extrémité fermée du dispositif a un diamètre d'environ 0,7 mm.

33. Dispositif chirurgical (10) selon la revendication 1, comportant un moyen pour dilater un orifice dans une partie du corps et un moyen destiné à s'engager dans un élément allongé, le moyen de dilatation comprenant le ballonnet gonflable à partir d'un état dégonflé (24) monté sur la partie de corps (12), le moyen d'engagement comprenant l'ouverture (22) sur la partie de corps, ladite ouverture étant adaptée pour recevoir le fil de suture (36), et le ballonnet pouvant être partiellement détaché de la partie de corps, de sorte à pouvoir être tiré au-dessus de l'ouverture.

34. Dispositif chirurgical selon la revendication 33, dans lequel la partie du corps est une gaine tendineuse (29).

35. Dispositif chirurgical selon les revendications 33 ou 34, dans lequel l'élément allongé est une structure flexible en forme de cordon (34).

36. Dispositif chirurgical selon la revendication 35, dans lequel la structure en forme de cordon est un tendon récupéré.

37. Dispositif chirurgical selon la revendication 35, dans lequel la structure en forme de cordon est une greffe de tendon.

38. Dispositif chirurgical selon la revendication 35, dans lequel la structure en forme de cordon est un matériau de tendon artificiel.

39. Dispositif chirurgical selon l'une quelconque des revendications 33 à 38, dans lequel le dispositif chirurgical est un dispositif chirurgical de dilatation et de passage.

40. Dispositif chirurgical selon l'une quelconque des revendications 33 à 39, dans lequel le dispositif chirurgical est un dispositif chirurgical de reconstruction d'un tendon.

41. Dispositif chirurgical selon l'une quelconque des revendications 33 à 40, dans lequel le ballonnet est monté sur la partie de corps au niveau de deux points de fixation, le ballonnet pouvant être détaché de la partie de corps au niveau du point de fixation le plus éloigné de l'ouverture.
